(19) Europäisches Patentamt
European Patent Office
Office européen des brevets

(11) Veröffentlichungsnummer: **0 057 812**
**B1**

(12) # EUROPÄISCHE PATENTSCHRIFT

(45) Veröffentlichungstag der Patentschrift: **27.06.84**

(51) Int. Cl.[3]: **C 07 C 103/52,** C 07 H 19/08, C 12 P 21/02, A 61 K 37/02 // (C12P21/02, C12R1/465)

(21) Anmeldenummer: **82100118.7**

(22) Anmeldetag: **11.01.82**

(54) **Neues Peptid-Antibiotikum Komponente A, Verfahren zu seiner Herstellung sowie seine Verwendung in Arzneimitteln.**

(30) Priorität: **23.01.81 DE 3102136**

(43) Veröffentlichungstag der Anmeldung: **18.08.82 Patentblatt 82/33**

(45) Bekanntmachung des Hinweises auf die Patenterteilung: **27.06.84 Patentblatt 84/26**

(84) Benannte Vertragsstaaten: **AT BE CH DE FR GB IT LI NL SE**

(56) Entgegenhaltungen:
**EP - A - 0 041 180**
**US - A - 4 158 608**

(73) Patentinhaber: **BAYER AG, Konzernverwaltung RP Patentabteilung, D-5090 Leverkusen 1 Bayerwerk (DE)**

(72) Erfinder: **Metzger, Karl Georg, Dr., Pahlkestrasse 15, D-5600 Wuppertal 1 (DE)**
Erfinder: **Pfitzner, Jörg, Dr., Claudiusweg 3, D-5600 Wuppertal 1 (DE)**
Erfinder: **Schmidt, Delf, Dr., Am Eckbusch 55b, D-5600 Wuppertal 1 (DE)**
Erfinder: **Weyland, Horst, Dr., Fichtestrasse 12, D-2850 Bremerhaven (DE)**
Erfinder: **Benz, Günter, Dr., Am Bölkumer Busch 5, D-5620 Velbert 15 (DE)**
Erfinder: **Schröder, Theo, Dr., Gellertweg 38, D-5600 Wuppertal 1 (DE)**

**EP 0 057 812 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die neue Verbindung Komponente A (Komponente B, vgl. EP-A-0 057 349), ein mikrobiologisches Verfahren zu ihrer Herstellung aus einem Streptomyces-Stamm sowie ihre Verwendung als Arzneimittel, insbesondere als antimikrobielles Mittel in der Human- und Tiermedizin.

Es ist bereits bekannt geworden, daß eine Reihe von Verbindungen mikrobieller Herkunft antimikrobielle Wirkungen besitzen. Diese Antibiotika sind teilweise in ihren Wirkungsspektren nicht voll befriedigend. Sie weisen häufig noch weitere Nachteile auf. β-Lactamantibiotika werden oft durch Penicillinase inaktiviert, Chloramphenicol, Tetracycline und Streptomycin zeigen in vielen Fällen erhebliche unerwünschte Nebenwirkungen (vgl. Walter, Heilmeyer, Antibiotika Fibel, Georg Thieme Verlag, Stuttgart, 3. Auflage, 1969, Seiten 248, 278—280 und 311—319).

Es wurde nun gefunden, daß man eine neue antibiotisch wirksame Verbindung erhält, wenn man den Stamm Streptomyces spec. WS 116 in einem Nährmedium züchtet und die Verbindung aus dem Nährmedium isoliert.

Die neue Verbindung weist starke antimikrobielle Wirkung auf. Sie kann eisenfrei oder eisenhaltig sein. Beide Formen können erfolgreich als antibakterielles Mittel eingesetzt werden.

Die neue Verbindung in der eisenfreien Form wird durch folgende Eigenschaften charakterisiert:

1. Die Elementaranalyse C 43,8%; H 6,7%; N 14,1%; O 30,3%; S 4,1%.
   Es muß hier darauf hingewiesen werden, daß bei höhermolekularen Naturstoffen die Fehlerbreite der Elementaranalyse größer sein kann als allgemein üblich, daher ist eine genaue Bestimmung der Summenformel oft nicht möglich (R. B. Woodward, Angew. Chem. 69, S. 50—51 [1957]).
2. Die gefriergetrocknete Verbindung schmilzt zwischen 180 und 185° C und zersetzt sich bei weiterer Erwärmung.
3. Ultraviolettabsorptionsspektrum:
   Das UV-Spektrum wurde an einer wäßrigen Lösung der Verbindung (c = 2.863 mg in 50 ml $H_2O$)aufgenommen. Die Spektren in saurer (bzw. basischer) Lösung wurden an einer Lösung gemessen, die durch Zusatz von 100 Mikrolitern 1 n Salzsäure (bzw. Natronlauge) zu 3 ml der obigen Lösung hergestellt wurde.

Tabelle 1

Maxima (λ max) und Extinktionen [$E^{1\%}_{1\,cm}$]der Verbindung

| | λmax [nm] | [$E^{1\%}_{1\,cm}$] |
|---|---|---|
| neutral | 267 | 103 |
| sauer | 267 | 97.10 |
| basisch | — | — |

4. Das IR-Absorptionsspektrum der Verbindung wird in Fig. 1 wiedergegeben (Abszisse: Wellenzahl in $cm^{-1}$, Ordinate: Absorption).
   Es zeigt, wenn die Substanz zu KBr-Preßlingen verpreßt wird, bei folgenden Wellenlängen (ausgedrückt in $cm^{-1}$) Absorptionsbanden:

Tabelle 2

Wellenlängen des IR-Absorptionsspektrums

| Wellenlänge in cm $^{-1}$ | Wellenlänge in cm $^{-1}$ |
|---|---|
| 3384 | 1390 |
| 2918 | 1300 |
| 1654 | 1240 |
| 1615 | 1210 |
| 1540 | 1160 |
| 1457 | 1090 |
| 1415 | 970 |

5. Das $^{1}$H-Kernresonanzspektrum gibt die Signallage in Teilen pro Million (ppm) und Schwingungen pro Sekunde gemäß Fig. 2 an. Es wurde an einer wäßrigen Lösung der Verbindung mit TMS-Na-Salz als Standard (extern) an einem WH-360 Spektrometer der Firma Bruker bei einer Feldstärke von 360 MHZ aufgenommen.
6. Das 13-C-Kernresonanzspektrum wurde an einem WM-250 Spektrometer der Fa. Bruker bei einer Feldstärke von 62,71 MHZ an einer wäßrigen Lösung der Verbindung aufgenommen, wobei auf Dioxan als externen Standard (Shiftlage 67 400 ppm rel. zu TMS = 0) umgerechnet wurde.
   Das 13-C-Kernresonanzspektrum gemäß Abbildung 3 zeigt die folgenden Signale angegeben in Teilen pro Million (ppm) und Schwingungen pro Sekunde (HZ) in ihren relativen Intensitäten an:

Tabelle 3

Shiftlagen und Intensitäten der Signale im 13-C-Kernresonanzspektrum, bezogen auf Dioxan = 67 400 ppm (extern)

| Signal NS | Rel. Intensität | Signallage (ppm) |
|---|---|---|
| 1 | 11 061 | 20 222 |
| 2 | 2 101 | 20 511 |
| 3 | 3 960 | 22 305 |
| 4 | 4 026 | 23 173 |
| 5 | 3 867 | 23 364 |
| 6 | 16 325 | 28 919 |
| 7 | 4 636 | 47 906 |
| 8 | 6 720 | 48 097 |
| 9 | 4 263 | 51 317 |
| 10 | 4 490 | 53 464 |
| 11 | 4 625 | 54 302 |
| 12 | 5 906 | 54 45 |
| 13 | 6 302 | 56 346 |

Fortsetzung

| Signal NS | Rel. Intensität | Signallage (ppm) |
|---|---|---|
| 14 | 4 260 | 58 317 |
| 15 | 5 171 | 61 993 |
| 16 | 4 332 | 64 581 |
| 17 | 5 260 | 70 022 |
| 18 | 5 100 | 75 962 |
| 19 | 5 436 | 80 536 |
| 20 | 5 437 | 102 328 |
| 21 | 5 790 | 142 604 |
| 22 | 4 421 | 153 765 |
| 23 | 5 004 | 166 249 |
| 24 | 3 694 | 170 322 |
| 25 | 4 663 | 171 337 |
| 26 | 3 740 | 174 028 |
| 27 | 3 837 | 174 395 |
| 28 | 5 046 | 174 778 |
| 29 | 2 662 | 174 925 |
| 30 | 5 686 | 175 792 |

7. Der optische Drehwert beträgt $[\alpha]$ = −20,216 (C = 0,3941% in Wasser).
8. Die Verbindung ist unbeschränkt in Wasser löslich, löst sich etwas in Methanol, DMF und DMSO und ist schwerlöslich in Chloroform, Äther, Essigester und Petroläther.
9. Die Verbindung ist ein farbloser, amorpher Feststoff, dessen wäßrige Lösung neutral reagiert.
10. Die $R_f$-Werte der Verbindung in der eisenfreien und eisenhaltigen Form im Vergleich mit anderen Verbindungen in verschiedenen Laufmitteln gibt Tabelle 4.
    a) DC-Fertigplatten Kieselgel 60 F 254 (Merck)
    Anfärbung: 1. Ninhydrin; 2. 5% $FeCl_3 \times 6\ H_2O$ in 0,5 n HCl
    Fließmittel 1 (FM 1): Isobutanol/Ethanol/Ammoniak = 9/1/5
    Fließmittel 2 (FM 2): Isobutanol/Ethanol/Ammoniak = 4/1/5
    10 cm Laufstrecke/Auftrag 50 µg in dest. Wasser

Tabelle 4a

| Substanz | kFM 1 | FM 2 |
|---|---|---|
| Neomycinsulfat | 0,01 | 0,16 |
| 2-Desoxystreptamin × 2 HCl | 0,03 | 0,19 |
| Sisomicin Base | 0,14 | 0,42 |
| erfindungsgemäße Verbindung, eisenfrei | 0,02 | 0,26 |
| dto., eisenhaltig | 0 | 0,23 |

b) DC-Fertigplatten Cellulose F (Merck)
Anfärbung: 1. Ninhydrin, 2. 5% $FeCl_3 \times 6\ H_2O$ in 0,5 n HCl
Fließmittel 1 (FM 1): 1-Butanol/Eisessig/dest. Wasser 4/1/5
Fließmittel 2 (FM 2): 1-Butanol/Eisessig/dest. Wasser 4/1/2
Fließmittel 3 (FM 3): 1-Propanol/Pyridin/Eisessig/dest. Wasser 15/10/3/12
10 cm Laufstrecke/Auftrag 50 µg in dest. Wasser.

Tabelle 4b

| Substanz | FM 1 | FM 2 | FM 3 |
|---|---|---|---|
| Neomycinsulfat | 0,06 | 0,06 | 0,06 |
| 2-Desoxystreptamine $\times$ 2 $H_2O$ | 0,10 | 0,13 | 0,42 |
| Sisomicin Base | 0,16 | 0,27 | 0,49 |
| erfindungsgemäße Verbindung, eisenfrei | 0,37 | 0,42 | 0,83 |
| dto., eisenhaltig | 0,14 | 0,25 | 0,74 |

11. Die neue Verbindung läßt sich in seiner eisenfreien Form auf der Dünnschichtplatte mit $FeCl_3$*) alk. Kaliumpermanganat, Jod, Ninhydrin und im UV-Licht bei 254 oder 280 nm durch Fluoreszenzlöschung sichtbar machen.

*) Die Sprühreagentien wurden nach den üblichen Vorschriften (z. B. E. Stahl, Dünnschichtchromatographie, 2. Auflage, Springer-Verlag, Berlin) hergestellt.

12. Die Totalhydrolyse der Verbindung mit wäßriger 57%iger Jodwasserstoffsäure zeigt bei der Aminosäureanalyse, wenn im verschlossenen Glas bei 110° C 24 Stunden hydrolysiert wird, daß die Substanz drei Einheiten Ornithin neben einer Einheit Serin enthält. Daneben wurden nur Spuren anderer natürlicher Aminosäuren gefunden.
Durch Derivatisierung, Isolierung von Hydrolyseprodukten und deren Derivaten konnte die Konstitution der erfindungsgemäßen Verbindung Komponente A aufgeklärt werden. Danach hat Komponente A folgende Struktur:

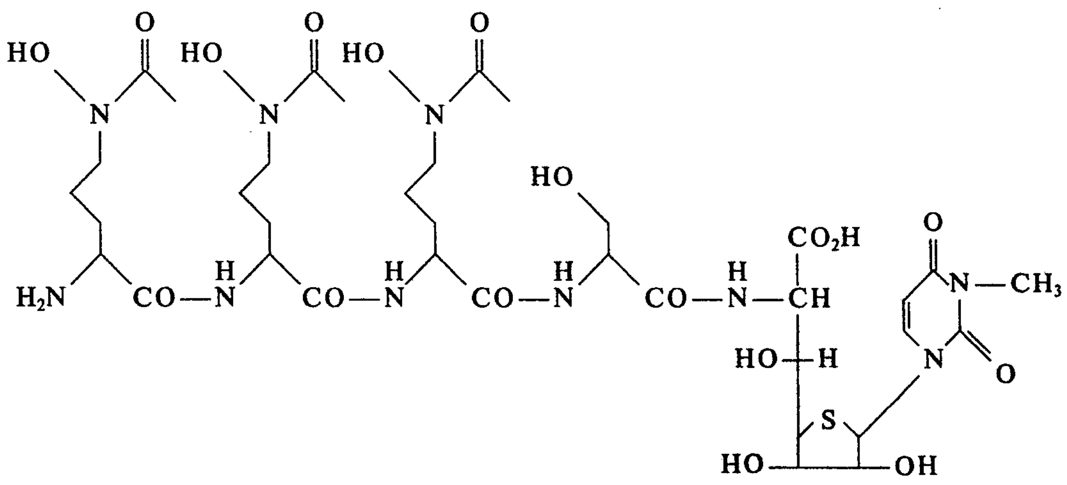

Die erfindungsgemäße Verbindung wird durch submerse Kultur eines Streptomyceten-Stammes in geeigneten Nährlösungen unter geeigneten physikalischen Bedingungen erzeugt. Sie wird aus der Kulturlösung durch Extraktion oder durch Adsorption abgetrennt und durch weitere geeignete Methoden angereichert.

Für das Herstellungsverfahren können der neue Stamm Streptomyces spec. WS 116 aus der Ordnung der Actinomycetales, Familie Streptomycetaceae, Gattung Streptomyces oder von diesem abstammende Varianten und Mutanten eingesetzt werden. Dieser Stamm wurde aus einer marinen Bodensedimentprobe der Ibero-Canarischen See isoliert. Er wurde unter der Nummer DSM 1692 am 7. 12. 1979 bei der Deutschen Sammlung für Mikroorganismen, Göttingen, hinterlegt.

a) Die Sporen sind ellipsoid. Sie haben die Größe von 0,4—0,7 × 0,7—1,2 µm und eine glatte Oberfläche.
b) Die Farbe des Luftmycels ist zu Anfang kreideweiß, im ausgereiften Zustand gelblich (Griseus-Typ).
c) Die Sporenketten sind gerade oder gewellt (Rectus-Flexibilis-Typ) und monopodial verzweigt.
d) Auf Pepton-Eisen-Agar sowie auf Tyrosin-Agar wurde kein schwarzbraunes Pigment gebildet. Der Stamm ist nicht chromogen.

Die zusammengefaßten Bestimmungsmerkmale identifizieren den Stamm WS-116 als zur Art Streptomyces griseus Waksman et Henrici gehörend.

Für das Verfahren zur Herstellung der erfindungsgemäßen Verbindung verwendet man Nährmedien, die die üblichen Kohlenstoff- und Stickstoffquellen und die notwendigen Salze enthalten. Als Kohlenstoffquelle können verwendet werden: Kohlenhydrate, insbesondere Polysaccharide, wie Stärke oder Dextrine, Disaccharide wie Maltose oder Rohrzucker, Monosaccharide, wie Glucose oder Xylose, Zuckeralkohole, wie Mannit oder Glycerin, Carbonsäuren, wie Citronensäure, Apfelsäure, Essigsäure oder deren Gemische, außerdem auch natürlich vorkommende Gemische, wie Malzextrakt. Überraschenderweise wurden mit Carbonsäuren, insbesondere mit Citronensäure als Haupt-C-Quelle die höchsten Wirkstoffausbeuten erzielt. Als Stickstoffquelle können die üblichen Stickstoffquellen Verwendung finden, so z. B. Eiweißstoffe, Eiweißhydrolysate, Aminosäuren wie Glutaminsäure, Asparaginsäure, Arginin, Lysin, Ornithin oder Serin, außerdem Nucleosidbasen wie Cytosin oder Uracil, Ammonium-Salze, Nitrate, natürlich vorkommende komplexe Stoffe, wie Peptone, Maisquellwasser, Sojabohnenmehl, Fleischextrakte, Hefeextrakte und geeignete Mischungen derselben. Besonders hohe Wirkstoffausbeuten erhält man, wenn zu den üblichen komplexen N-Quellen L-Ornithin und L-Serin im Verhältnis 3 : 1, z. B. 0,3% und 0,1%, in sterilfiltrierter Form dem Medium zugesetzt werden.

Als Hilfsstoffe werden im Nährmedium Mineralsalze benötigt, z. B. Phosphate, Sulfate, Carbonate, Nitrate oder Chloride des Natriums, Kaliums, Calciums, Magnesiums, Eisens, Zinks, Kupfers, Molybdäns, Kobalts, Nickels und Mangans. Als bedeutsam erwies sich die Anwesenheit von etwa 0,01% $FeCl_3$. Zum Teil sind die Mineralsalze, auch das $FeCl_3$, in den notwendigen Konzentrationen in den oben genannten Kohlenstoff- oder Stickstoffquellen oder im verwendeten Wasser als Bestandteile enthalten.

Weiterhin können als Hilfsstoffe noch Antischaummittel der verschiedensten Art Verwendung finden, wie z. B. Sojaöl, Polyole oder Silikone.

Als wichtigstes Verdünnungsmittel für die Nährmedien ist Wasser zu nennen.

Bei der Durchführung des Herstellungsverfahrens wird unter aeroben bzw. mikroaerophilen Bedingungen gearbeitet; die Kultur kann gemäß üblicher Methoden, so z. B. unter Verwendung von Schüttelkulturen oder von belüfteten Fermenterkulturen im üblichen Batch- oder Fedbatch-Verfahren, durchgeführt werden. Die Prozentverhältnisse (jeweils Gewichtsprozente) der Nährlösungsbestandteile können in weiten Bereichen schwanken, im allgemeinen machen die Kohlenstoffquellen insgesamt 0,5—8%, vorzugsweise 0,6—6% aus, die Stickstoffquellen insgesamt 0,05 bis 4%, vorzugsweise 0,5 bis 2% aus; die Salze liegen in üblichen Konzentrationen vor, vorzugsweise im Bereich zwischen 0,001 bis 0,5 Gew.-%. Die Antischaummittel liegen in 0- bis 1%iger Konzentration vor. Die zur Sterilisation angewandten Temperaturen liegen bei 100 bis 140°C, vorzugsweise bei 120 bis 130°C, empfindliche Substanzen wie Aminosäuren werden sterilfiltriert.

Die pH-Werte der wachsenden Kulturen liegen zwischen 5 und 10, vorzugsweise zwischen 6 und 9,5. Die Züchtungstemperatur kann zwischen 15 und 35°C, vorzugsweise zwischen 20 und 30°C liegen. Es hat sich herausgestellt, daß die Menge des sich in der Kulturbrühe anreichernden Antibioticums im allgemeinen ihr Maximum etwa 1 bis 10, vorzugsweise etwa 2 bis 6 Tage nach Züchtungsbeginn erreicht. Der Endpunkt der Fermentation wird mit Hilfe von biologischen Tests ermittelt (Wirkung gegen E. coli in einem üblichen Agardiffusionstest).

Die erfindungsgemäße Verbindung wird aus dem Kulturfiltrat durch Extraktion mit Mischungen aus Phenol/Chloroform oder aber durch Adsorption an Aktivkohle bzw. an geeignete Harze isoliert. Vorteilhaft ist die Bindung der erfindungsgemäßen Verbindung an unspezifische Adsorberharze auf Polystyrolbasis (z. B. Amberlite® XAD der Fa. Rohm & Haas oder Lewatit® OC 1031 der Fa. Bayer). Überraschenderweise wurde gefunden, daß die erfindungsgemäße Verbindung besonders fest von derartigen Harzen gebunden wird, wenn man vor dem Adsorptionsvorgang Eisensalze, insbesondere Eisenchlorid in Konzentration von 0,05 bis 0,2, insbesondere ca. 0,1 g/Liter Kulturbrühe, hinzugibt. Die Adsorption führt man im pH-Bereich 3—9, insbesondere im Bereich 5—7, durch. Die Desorption der erfindungsgemäßen Verbindung wird fraktioniert durch Mischungen aus Wasser und organischen Lösungsmitteln, insbesondere Wasser/Methanol, vorgenommen. Die aktiven Fraktionen werden vereinigt, auf ein kleines Volumen eingeengt und lyophilisiert. Man erhält ein 0,5—3%iges Rohprodukt, das die erfindungsgemäße Verbindung neben anderen Substanzen enthält.

Ausgehend von diesem Rohprodukt kann die weitere Anreicherung der erfindungsgemäßen Verbindung durch eine Kombination von Anionen- (z. B. DEAE-Sephadex® A 25, Fa. Pharmacia) bzw. Kationen-Austauschchromatografie (z. B. SP- oder CM-Sephadex® C 25, Fa. Pharmacia) durchgeführt werden. Man erhält dadurch ein 30—50%iges Präparat, da begleitende Peptide nicht abgetrennt werden.

Die Abtrennung dieser Peptide und damit die Darstellung der reinen Verbindung gelingt durch Adsorptions- bzw. Verteilungschromatografie der ca. 30–50%igen Verbindung an Kieselgel im System Isobutanol/Ethanol/25% konz. Ammoniak = 9/1/5 (Volumenteile).

Dieses Trennverfahren ist jedoch mit Substanzverlusten verbunden.

Wesentlich einfacher gelingt die Trennung durch Affinitätschromatografie in einer $Fe^{+++}$-haltigen Säule. Dazu überführt man einen Kationenaustauscher auf Polystyrolharz- oder Acrylharzbasis (z. B. Dowex® 50 WX 4, Fa. DOW Chemical) oder auf Polydextranbasis (z. B. Sephadex® C 25, Fa. Pharmacia) mit $FeCl_3$-Lösung in die $Fe^{+++}$-Form. Auf das Harz in der $Fe^{+++}$-Form wird nun die Lösung des Rohproduktes aufgetragen und anschließend mit Wasser nachgewaschen. Dann eluiert man mit einem Puffer hoher Ionenstärke, z. B. 0,2 m $NaH_2PO_4$/0,3 m NaCl. Dieser Puffer eluiert die Hauptmenge der inaktiven Begleitpeptide. Anschließend eluiert man die wirksamen Substanzen von der Säule mit dem gleichen Puffer, jedoch unter Zusatz von 0,05 m Ethylendiamintetraessigsäure oder einem anderen Eisenkomplexbildner (z. B. Citrat). Die aktiven Fraktionen werden vereinigt und über eine Säule mit unspezifischen Adsorptionsharz (z. B. Lewatit® OC 1031, Fa. Bayer) gegeben, wobei die wirksamen Substanzen gebunden werden. Man eluiert mit Methanol, engt ein und lyophilisiert.

Die erfindungsgemäße Verbindung wird durch Verteilungschromatografie an Sephadex® G 25 in n-BuOH/iso BuOH/0,2 m $(NH_4)_2SO_4$ = 2/1/1 rein erhalten. Die Reindarstellung des erfindungsgemäßen Produktes wird ferner durch einfache Chromatografie des Wirkstoffgemisches an einer CM-Cellulose-Säule in der $H^+$-Form mit dest. Wasser ohne irgendwelche Zusätze durchgeführt. Das Eluat, in Fraktionen aufgefangen, wird lyophilisiert.

Die gute antimikrobielle Wirksamkeit der erfindungsgemäßen Verbindung wird durch In-vitro- und In-vivo-Versuche demonstriert.

In vitro wird im Agar-Verdünnungstest nach dem international üblichen Test (American National Committee for Climical Laboratory Standards = NCCLS) Wirksamkeit gegen Enterobacteriaceae, z. B. coli 14 und E. coli C 165 gefunden.

In vivo wird an der weißen Maus eine $ED_{100}$ (vollständige Heilung) bei Infizierung mit E. Coli Neumann bei 3 mg Antibiotikum/kg Körpergewicht gefunden; Therapie: einmalig 30 Minuten nach Infektion subcutan.

Die neue Verbindung Komponente A kann in üblichen pharmazeutischen Zusammensetzungen angewendet werden. Die pharmazeutischen Zubereitungen enthalten den erfindungsgemäßen Wirkstoff und übliche geeignete, nicht-toxische Träger- und Hilfsstoffe.

Geeignete Zubereitungen und die dazu notwendigen Hilfs- und Trägerstoffe sind beispielsweise aus der Deutschen Offenlegungsschrift 25 10 161 bekannt.

Komponente A (eisenhaltig)

| Keim | MHK in µg/ml |
|---|---|
| Escherichia coli F14 | 0,1 |
| Escherichia coli 183/58 | 0,1 |
| Escherichia coli 14 | 0,1 |

Die Bestimmung der minimalen Hemmkonzentration (MHK) erfolgte mit einem Inoculator im Agarverdünnungsverfahren mit einer Einsaatdichte von $10^4$ pro Impf-Punkt. Die MHK ist die Konzentration, bei der keine Bakterienkolonien wachsen.

Komponente A (eisenhaltig) Tierversuch (Mäuse)

| Infektionskeim | $ED_{100}$ nach intraperitonealer Infektion und subcutaner Behandlung in mg/kg |
|---|---|
| E. coli Neumann | 0,5 |
| E. coli 205 Kn | 0,1 |
| Klebsiella pneumoniae 63 | 0,2 |
| Staphylococcus aureus 133 | 0,6 |

Die Tiere waren mit einer Bakterienmenge infiziert, welche innerhalb von 24 Stunden zum Tod der Kontrolltiere führte. Die $ED_{100}$ ist die Dosis, die alle infizierten und behandelten Tiere überleben läßt.

Beispiel 1a)

Die Nährlösung, in der der Produktionsstamm Streptomyces spec. WS 116 in den Vorkulturen kultiviert wird, setzt sich aus 1 Gew.-% Glucose, 1,3% Hefeextrakt, 0,05% Polyol und Leitungswasser zusammen. Der pH wird vor dem Sterilisieren auf 7,0 eingestellt. 4 × 1000-ml-Erlenmeyerkolben, die je 150 ml dieser Nährlösung enthalten, werden mit dem Produktionsstamm beimpft und 4 Tage bei 28°C auf einer Rundschüttelmaschine bei 220 Umdrehungen/min inkubiert. Mit diesen Vorkulturen wird eine zweite Vorkultur in einem Labor-Fermenter, der 20 l der oben erwähnten Nährlösung enthält, beimpft und bei 200 Umdrehungen/min, 10 l Luft/min und 28°C 2 Tage inkubiert. Mit 20 l dieser Kultur wird ein Produktionsfermenter beimpft, der 600 l Nährlösung mit folgender Zusammensetzung enthält: 0,7 Gew.-% Citronensäure, 0,8% Hefeextrakt, 0,2% entfettetes Sojabohnenmehl, 0,2% Maisquellwasser und 0,05% Silicon in Leitungswasser. Der pH dieser Nährlösung wird mit Kalilauge auf 6,4 vor der Sterilisation eingestellt. Die Inkubation der Produktionskultur erfolgt über 2—4 Tage bei 26°C bei einer Rührung von 50 Umdrehungen/min und einer Belüftung von nur 90 l Luft/min. Die Fermentation wird bei optimaler antibiotischer Hemmaktivität der Kultur abgestoppt.

Beispiel 1b)

2 × 150 ml Vorkultur werden, wie im Beispiel 1a) angegeben, angezogen. Mit diesen Vorkulturen wird ein 10-l-Produktionsfermenter beimpft, dessen Nährlösung, in Leitungswasser angesetzt, folgende Zusammensetzung enthält:

| | |
|---|---|
| 0,7 Gew.-% | Citronensäure, |
| 0,8 Gew.-% | Hefeextrakt, |
| 0,2 Gew.-% | entfettetes Sojabohnenmehl, |
| 0,2 Gew.-% | Maisquellwasser, |
| 0,3 Gew.-% | L-Ornithin, |
| 0,1 Gew.-% | L-Serin und |
| 0,05 Gew.-% | Silicon. |

Alle Bestandteile außer Ornithin und Serin werden wie üblich im Kulturgefäß sterilisiert. Es wird ein pH-Wert von 6,4 vor der Sterilisation eingestellt. Ornithin und Serin werden in $H_2O$ dest. gelöst dem Ansatz sterilfiltriert zugesetzt.

Die Inkubation der Produktionskultur erfolgt über 2—4 Tage bei 26° mit einer Rührung von 200 Umdrehungen/min und einer Belüftung von nur 1,5 l Luft/min. Die Fermentation wird bei optimaler antibiotischer Hemmaktivität des Kulturüberstandes abgestoppt.

Beispiel 2

4000 l Kulturbrühe (pH = 9,06) wurden mit 50 l 1 : 1 verdünnter HCl auf pH 6,2 eingestellt. Man setzte 400 g $FeCl_3 \cdot 6\, H_2O$ hinzu, rührte und gab dann 25 l verdünnte NaOH bis pH 7 unter Rühren hinzu. Dann wurde mit 200—250 l/h im Westfalia-Separator separiert. Der Überstand wurde durch eine 30 × 70 cm hoch mit Lewatit® OC 1031 (= unsp. Adsorptionsharz der BAYER AG) gefüllte Säule gegeben, der Durchlauf kanalisiert, da inaktiv. Die Säule wurde mit 1000 l entionisiertem Wasser gewaschen, das Waschwasser war inaktiv und wurde verworfen. Die Säule wurde nun mit 1000 l 15% Methanol gewaschen, dieses inaktive Waschwasser wurde ebenfalls verworfen. Man eluierte die Aktivität nun mit 50% Methanol von der Säule und sammelte 100-l-Fraktionen. Die aktiven Eluate 2 und 3 wurden vereinigt, eingeengt im Dünnschichtverdampfer auf ca. 20 l und dann lyophilisiert. Man erhielt 342 g Rohprodukt der erfindungsgemäßen Verbindung mit einem Gehalt von ca. 2,5% (Komponenten A und B; Komponente B vgl. EP-A-0 057 349).

Beispiel 3

Man löste die obige Rohsubstanz in 6 Litern $H_2O$ und versetzte unter Rühren mit 25 ml 50%iger $FeCl_3$-Lösung. Der sich bildende Niederschlag wurde nach 15 Minuten Rühren abzentrifugiert (Hettich Rota Magna Zentrifuge, 1,5 l Becher, 30 min, 4000 Upm). Der Überstand wurde mit Schwerkraft über eine 8 × 45 cm hoch mit SP-Sephadex® C 25 $Fe^{+++}$ gefüllte Säule gegeben. Die Fließrate betrug 4 l pro Stunde. Man wusch die schwarz gefärbte Säule mit 5 l dest. $H_2O$ nach und schloß dann 10 l 0,2 m

$NaH_2PO_4$/0,3 NaCl Puffer an. Durchlauf und Waschwasser enthielten weniger als 5% der eingesetzten antibiotischen Aktivität. Die jetzt nur noch hellbraun gefärbte Säule wurde nun mit 0,2 m $NaH_2PO_4$/0,3 m NaCl/0,05 m AeDTA eluiert (Fließrate 2—3 l/h), das Säuleneluat fraktioniert in 500-ml-Portionen aufgefangen. Die aktiven Fraktionen 6—14 wurden vereinigt und über eine 5 × 40 mit Lewatit® OC 1031 gefüllte Säule gegeben. Die Fließrate betrug 3 l/h. Man wusch anschließend mit destilliertem Wasser bis mit $AgNO_3$ keine Cl im Säuleneluat nachzuweisen war (ca. 6 Liter, Fließrate 5 l/h). Anschließend eluierte man die Säule mit 3 Liter 90% Methanol, die im batch aufgefangen, eingeengt und lyophilisiert wurden. Ausbeute: 6,74 g, 82,6% Komponente A und B.

Beispiel 4

Die halbe Ausbeute aus Beispiel 2 = 3,37 g wurden in 100 ml dest. $H_2O$ gelöst. Die Leitfähigkeit betrug 210 µS.

Diese Lösung wurde auf eine 5 × 30 cm mit CM-Cellulose in der $H^+$-Form (CM-Cellulose C 52, Fa. Whatmann) gefüllte Säule aufgetragen. Man entwickelte mit dest. Wasser bei einer Fließrate von 840 ml/h. Das Eluat wurde an Hand der Refraktions-, Leitfähigkeits und Extraktionskurve geschnitten. Man erhielt Vorlauf (inaktiv) 980 mg

| | |
|---|---|
| Fraktion 1 | 427 mg |
| Fraktion 2 | 674 mg |
| Fraktion 3 | 363 mg |
| Fraktion 4—6 | 475 mg |
| Fraktion 7 | 253 mg |
| Fraktion 8 | 96 mg |

Fraktion 1 ist die erfindungsgemäße Verbindung Komponente A.

**Patentansprüche**

1. Verbindung der allgemeinen Formel

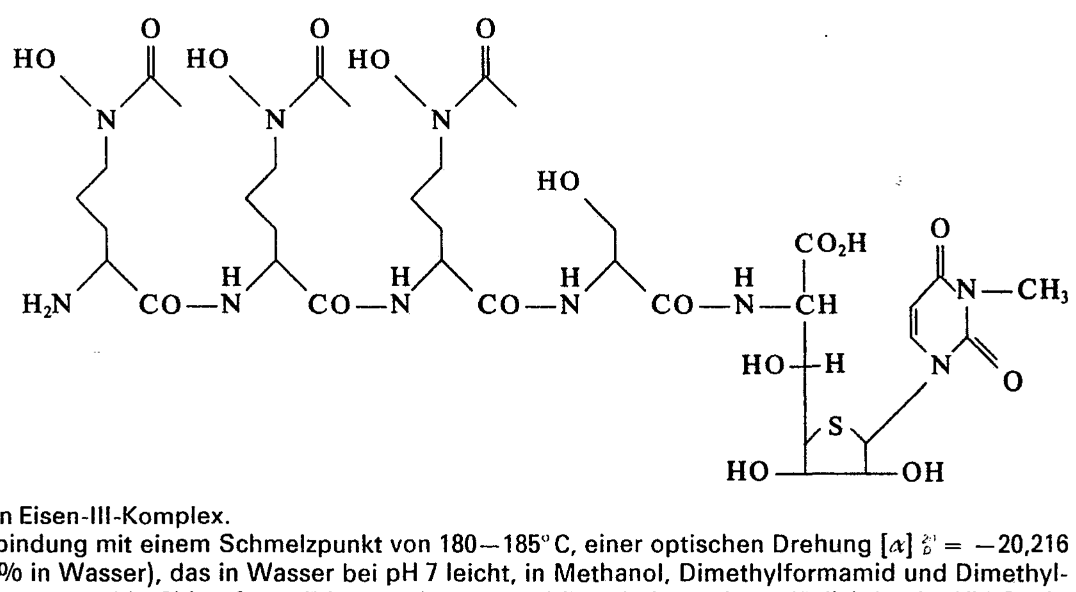

und deren Eisen-III-Komplex.

2. Verbindung mit einem Schmelzpunkt von 180—185° C, einer optischen Drehung $[\alpha]_D^{21}$ = —20,216 (c 0,3941% in Wasser), das in Wasser bei pH 7 leicht, in Methanol, Dimethylformamid und Dimethylsulfoxid etwas und in Chloroform, Ether, Essigester und Petrolether schwer löslich ist, im UV-Spektrum in Wasser (neutral und sauer) ein Maximum bei 267 nm, als KBr-Preßling im infraroten Bereich Absorptionsmaxima bei 3348, 2918, 1654, 1615, 1540, 1457, 1415, 1390, 1300, 1240, 1210, 1160, 1090 und 970 $cm^{-1}$ aufweist und bei saurer Hydrolyse Ornithin und Serin im Verhältnis 3 : 1 freisetzt, und deren Eisen-III-Komplex.

3. Antibiotikum gemäß Anspruch 1 oder 2, gekennzeichnet durch das 1 H-NMR-Spektrum gemäß Fig. 2, durch 13 C-NMR-Spektrum gemäß Fig. 3 und durch ein Maximum bei 267 nm im UV-Spektrum.

4. Verfahren zur Herstellung der Verbindung gemäß den Ansprüchen 1 bis 3, dadurch gekennzeichnet, daß man unter submersen, aeroben Bedingungen Streptomyces spec. WS 116 (DSM 1692) in einem assimilierbaren Kohlenstoff, Stickstoff sowie Mineralsalze, insbesondere Eisensalze enthaltenden Nährmedium bei einer Temperatur von 15 bis 35° C züchtet und die gebildete Verbindung aus der Fermentationsflüssigkeit isoliert.

5. Verfahren nach Anspruch 4, dadurch gekennzeichnet, daß man zur Isolierung die erfindungsgemäße Verbindung an ein unspezifisches Adsorptionsharz bindet und mit Mischungen aus Wasser und organischem Lösungsmittel desorbiert.

6. Verfahren nach den Ansprüchen 4 bis 5, dadurch gekennzeichnet, daß man die Kulturbrühe vor der Adsorption mit einem Eisensalz versetzt.

7. Verfahren nach den Ansprüchen 4 bis 6, dadurch gekennzeichnet, daß man zur Isolierung die rohe Verbindung über einen Kationenaustauscher in der $Fe^{3+}$-Form chromatographiert, inaktive Begleitstoffe mit einem Puffer hoher Ionenstärke eluiert und die Verbindung mit dem gleichen Puffer unter Zusatz eines Eisen-Komplexbildners eluiert.

8. Verfahren nach Anspruch 7, dadurch gekennzeichnet, daß man das nach Anspruch 7 erhaltene Produkt über eine CM-Cellulose-Säule in der $H^+$-Form chromatographiert und mit Wasser fraktioniert eluiert.

9. Arzneimittel mit einem Gehalt an der Verbindung gemäß einem der Ansprüche 1 bis 3.

10. Verbindung gemäß einem der Ansprüche 1 bis 3 zur Verwendung bei der Bekämpfung bakterieller Infektionen.

11. Verfahren nach den Ansprüchen 4 bis 8, dadurch gekennzeichnet, daß in einer Nährlösung fermentiert wird, die als Hauptkohlenstoffquelle Citronensäure, mehrere komplexe Stickstoffquellen und die Aminosäuren L-Ornithin und L-Serin enthält.

12. Verfahren nach den Ansprüchen 4 bis 8, dadurch gekennzeichnet, daß in einer vollsynthetischen Nährlösung fermentiert wird, die neben Mineralsalzen als Kohlenstoffquelle Citronensäure und als einzige Stickstoffquelle L-Arginin enthält.

13. Verfahren nach den Ansprüchen 4 bis 8, dadurch gekennzeichnet, daß zu einer Nährlösung zur Ausbeutesteigerung ein Gemisch von Citronensäure-L-Arginin und Salzen in solchen Raten nachgefüttert wird, daß ein konstanter pH-Wert aufrechterhalten wird.

**Claims**

1. Compound of the general formula

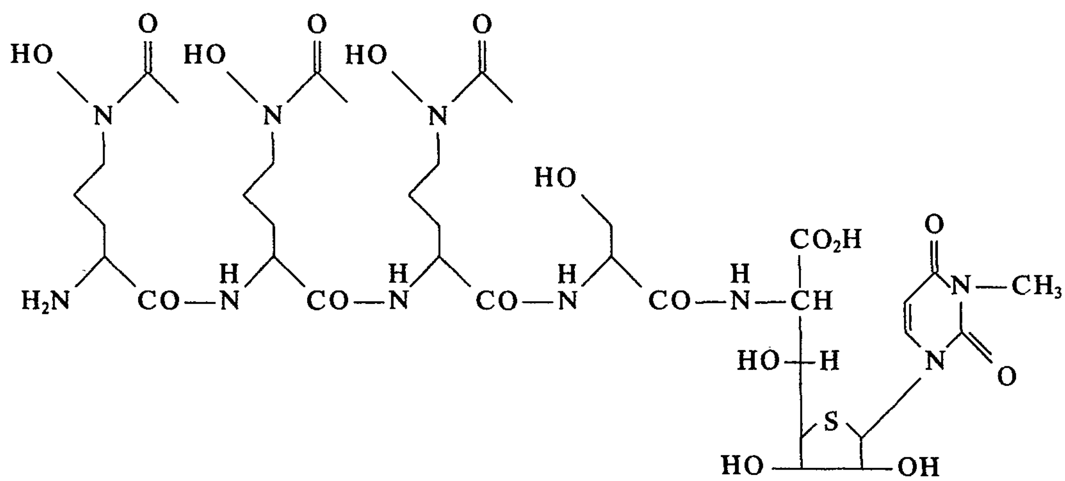

and its iron (III) complex.

2. Compound with a melting point of 180—185° C and an optical rotation $[\alpha]^{20}_{D} = -20.216$ (c 0.3941% in water), which is readily soluble in water at pH 7, slightly soluble in methanol, dimethylformamide and dimethylsulphoxide, and sparingly soluble in chloroform, ether, ethyl acetate and petroleum ether, has a maximum at 267 nm in the UV spectrum in water (neutral and acid), and, as a KBr pressed plate, has absorption maxima at 3348, 2918, 1654, 1615, 1540, 1457, 1415, 1390, 1300, 1240, 1210, 1160, 1090 and 970 $cm^{-1}$ in the infrared range, and releases ornithine and serine in the ratio 3 : 1 on acid hydrolysis, and its iron (III) complex.

3. Antibiotic according to Claim 1 or 2, characterised by the 1 H-NMR spectrum according to Fig. 2, by the 13 C-NMR spectrum according to Fig. 3 and by a maximum at 267 nm in the UV spectrum.

4. Process for the preparation of the compound according to Claims 1 to 3, characterised in that Streptomyces spec. WS 116 (DSM 1692) is grown, under submerse, aerobic conditions, in a nutrient medium containing assimilable carbon, nitrogen and mineral salts, in particular iron salts, at a temperature of from 15 to 35° C, and the compound formed is isolated from the fermentation liquid.

5. Process according to Claim 4, characterised in that the compound according to the invention is isolated by binding it to a non-specific adsorption resin and desorbing it with mixtures of water and an organic solvent.

6. Process according to Claims 4 to 5, characterised in that an iron salt is added, before the

adsorption, to the culture broth.

7. Process according to Claims 4 to 6, characterised in that the crude compound is isolated by subjecting it to chromatography over a cation exchanger in the $Fe^{3+}$ form, eluting inactive accompanying substances with a buffer of high ionic strength, and eluting the compound with the same buffer with the addition of an iron complex-forming agent.

8. Process according to Claim 7, characterised in that the product obtained according to Claim 7 is subjected to chromatography over a CM-cellulose column in $H^+$ form and is fractionally eluted with water.

9. A medicament containing the compound according to one of Claims 1 to 3.

10. Compound according to one of Claims 1 to 3 for use in combating bacterial infections.

11. Process according to Claims 4 to 8, characterised in that the fermentation is carried out in a nutrient solution which contains citric acid as the main carbon source, several complex nitrogen sources, and the aminoacids L-ornithine and L-serine.

12. Process according to Claims 4 to 8, characterised in that the fermentation is carried out in a completely synthetic nutrient solution which, in addition to mineral salts, contains citric acid as the carbon source and L-arginine as the only nitrogen source.

13. Process according to Claims 4 to 8, characterised in that, in order to increase the yield, a mixture of citric acid, L-arginine and salts is further fed to a nutrient solution at such rates that a constant pH value is maintained.

## Revendications

1. Composé de formule générale

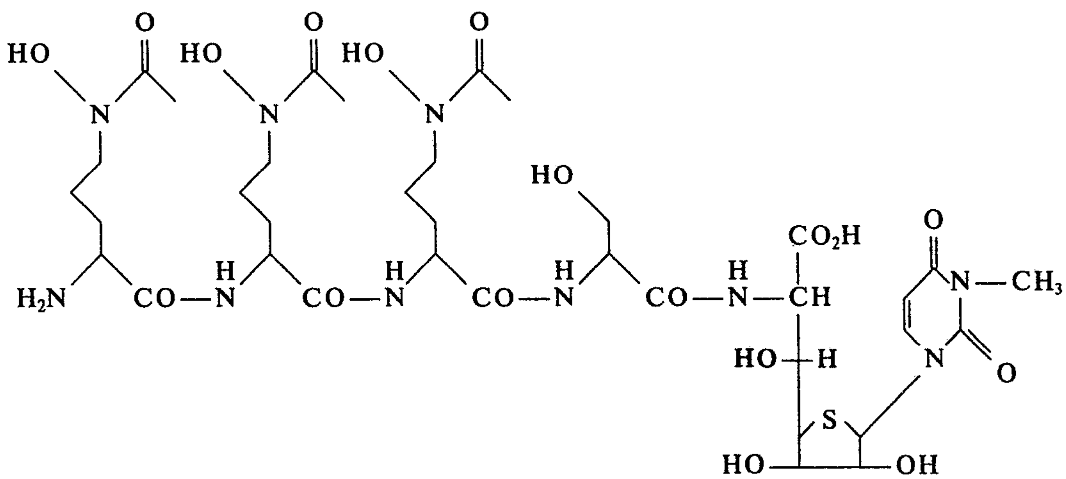

et son complexe de fer III.

2. Composé ayant un point de fusion de 180—185°C, une rotation optique $[\alpha]$ = −20,216 (c = 0,3941% dans l'eau), légèrement soluble dans l'eau à pH 7, un peu soluble dans le méthanol, le diméthylformamide et le diméthylsulfoxyde et peu soluble dans le chloroforme, l'éther, l'acétate d'éthyle et l'éther de pétrole, ayant dans le spectre UV dans l'eau (neutre et acide) un maximum à 267 nm, des maxima d'absorption dans l'infrarouge en pastille de KBr à 3348, 2918, 1654, 1615, 1540, 1457, 1415, 1390, 1300, 1240, 1210, 1160, 1090 et 970 $cm^{-1}$ et libérant par hydrolyse acide l'ornithine et la sérine dans le rapport 3 : 1, et son complexe de fer III.

3. Antibiotique selon la revendication 1 ou 2, caracterisé par le spectre de RMN de 1 H selon la fig. 2, par le spectre de RMN de 13 C selon la fig. 3 et par un maximum A 267 nanometre dans le spectre UV.

4. Procédé pour la fabrication du composé selon les revendications 1 à 3, caracterisé en ce que l'on cultive en conditions aérobies submergées Streptomyces spec. WS 116 (DSM 1692) dans un milieu nutritif contenant du carbone et de l'azote assimilables et des sels minéraux, en particulier des sels de fer, à une température de 15 à 35° C, et on isole le composé formé du liquide de fermentation.

5. Procédé selon la revendication 4, caracterisé en ce que l'on fixe pour l'isolement le composé selon l'invention sur une résine d'adsorption non spécifique et on le désorbe par des mélanges d'eau et de solvant organique.

6. Procédé selon les revendications 4 et 5, caracterisé en ce que l'on ajoute un sel de fer au bouillon de culture avant l'adsorption.

7. Procédé selon les revendications 4 à 6, caracterisé en ce que l'on chromatographie pour l'isolement le produit brut sur un échangeur de cations sous la forme $Fe^{3+}$, on élue les substances inactives qui l'accompagnent avec un tampon de force ionique élevée et on élue le composé avec le même tampon avec additon d'un agent complexant du fer.

8. Procédé selon la revendication 7, caractérisé en ce que l'on chromatographie le produit obtenu selon la revendication 6 sur une colonne de CM-Cellulose sous la forme $H^+$ et on effectue son élution fractionnée par l'eau.

9. Médicament contenant le composé selon l'une des revendications 1 à 3.

10. Composé selon l'une des revendications 1 à 3, pour l'utilisation dans la lutte contre les affections bactériennes.

11. Procédé selon les revendications 4 à 8, caracterisé en ce que l'on effectue la fermentation dans une solution nutritive qui contient de l'acide citrique comme principal source de carbone, plusieurs sources d'azote complexes et les aminoacides L-ornithine et L-sérine.

12. Procédé selon les revendications 4 à 8, caracterisé en ce que l'on effectue la fermentation dans une solution nutritive totalement synthétique, qui contient outre les sels minéraux de l'acide citrique comme source de carbone et comme seule source d'azote de la L-arginine.

13. Procédé selon les revendications 4 à 8, caracterisé en ce que, pour l'augmentation du rendement, on ajoute à une solution nutritive un mélange d'acide citrique, de L-arginine et de sels à des taux tels que l'on maintienne un pH constant.

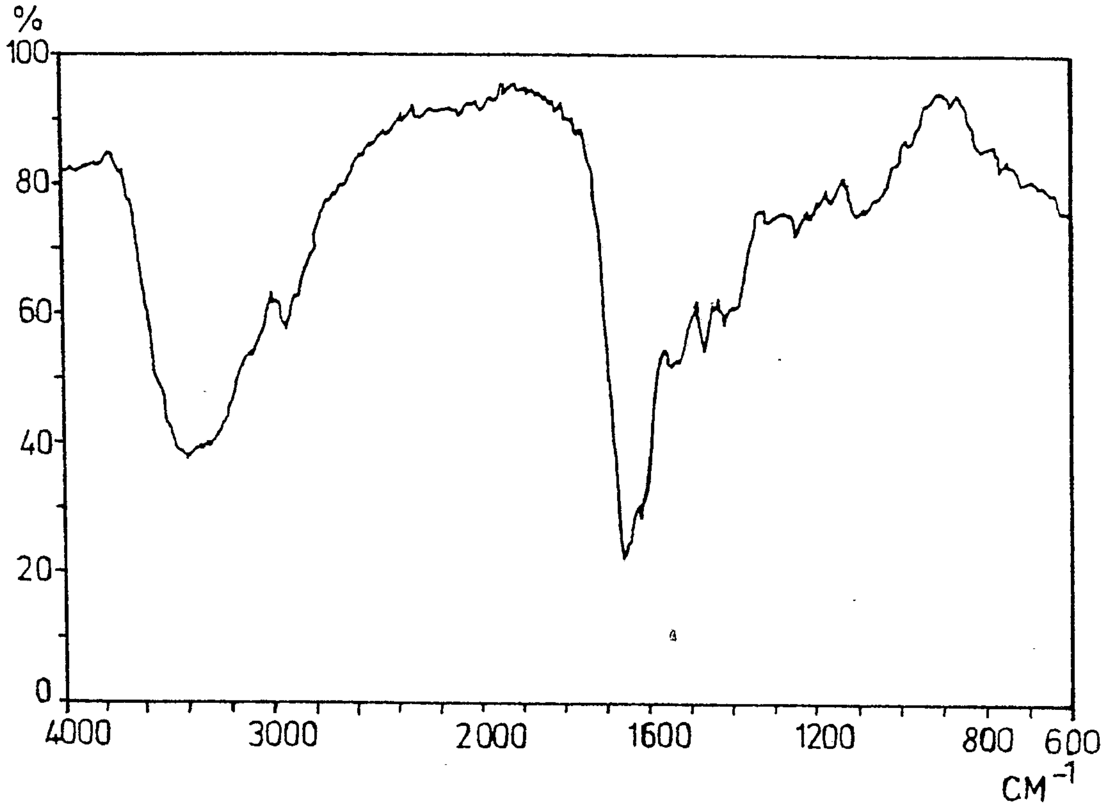


FIG. 1

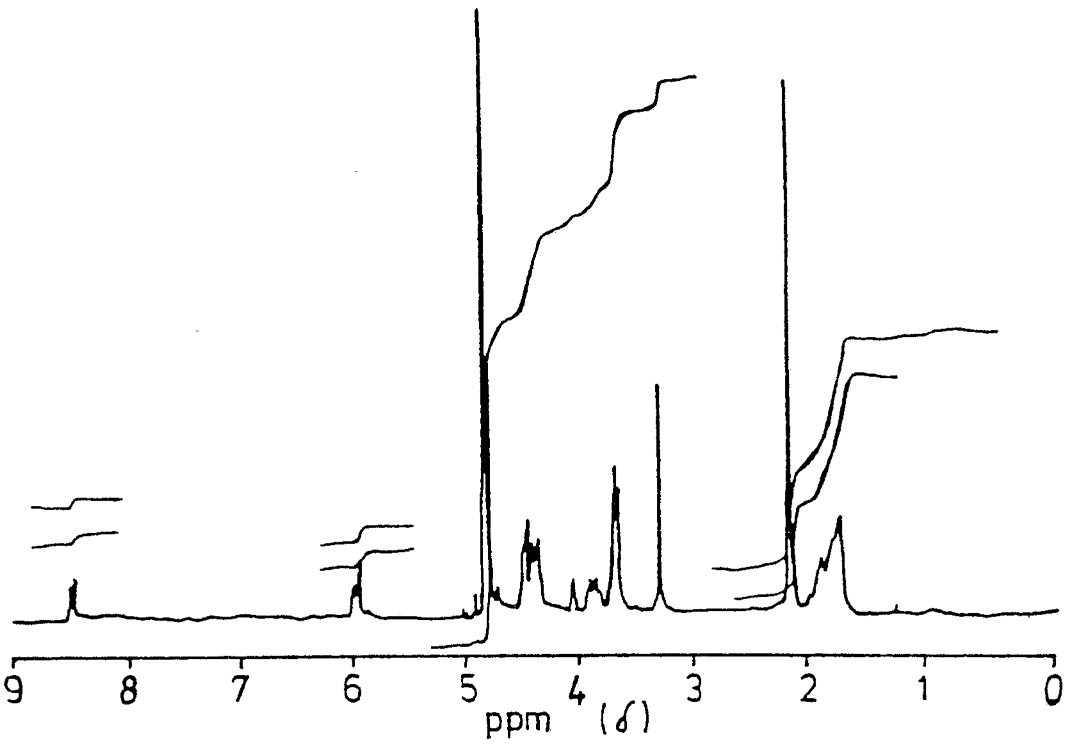


FIG. 2

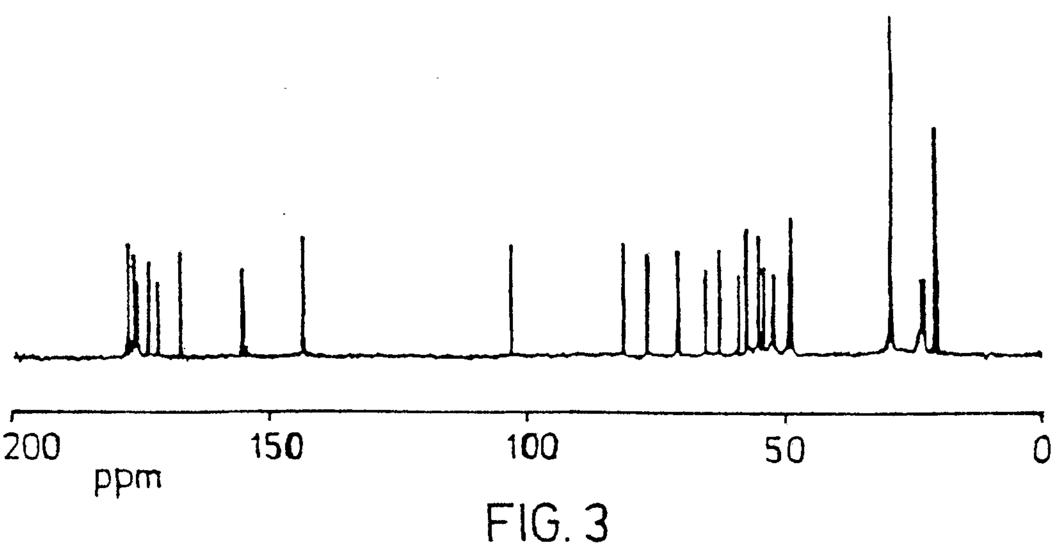


FIG. 3